# EUROPEAN PATENT APPLICATION

(11) **EP 3 395 249 A1**
(43) Date of publication of application: **31.10.2018**
(21) Application number: 16878944.4
(22) Date of filing: 22.12.2016
(51) Int. Cl.: A61B 5/11, G01B 7/16

(54) **SWALLOWING MOVEMENT MEASURING DEVICE AND SWALLOWING MOVEMENT MEASURING METHOD**

(30) Priority: 24.12.2015 JP 2015252471
(71) Applicant: Bando Chemical Industries, Ltd., Kobe-shi, Hyogo 650-0047 (JP)
(72) Inventor: OHTA, Masashi, Kobe-shi Hyogo 650-0047 (JP); OTAKA, Hideo, Kobe-shi Hyogo 650-0047 (JP); BESSHO, Yusuke, Kobe-shi Hyogo 650-0047 (JP); ISHIKAWA, Akira, Kobe-shi Hyogo 657-8501 (JP); YAMAMOTO, Akio, Kobe-shi Hyogo 657-8501 (JP); HANAIE, Kaoru, Kobe-shi Hyogo 657-8501 (JP); YAMAGUCHI, Takumi, Kobe-shi Hyogo 657-8501 (JP); UMEHARA, Ken, Kobe-shi Hyogo 657-8501 (JP)
(74) Representative: Sajda, Wolf E.
(86) International application number: PCT/JP2016/088448
(87) International publication number: WO 2017/111058

(57) **Abstract**

Provided is a swallowing movement measuring device capable of an examinee's swallowing movement. This swallowing movement measuring device is a device for measuring an examinee's swallowing movement on the basis of a movement of the examinee's larynx when the examinee swallows a food bolus. This device has a capacitive sensor to be brought into contact with a body skin of the examinee's laryngeal part, and a measuring unit that measures a change of the capacitive sensor in capacitance. This capacitive sensor includes a sensor body comprising sheet-like dielectric layer, a first electrode layer and a second electrode layer, the first and the second electrode layers are formed on a top surface and a bottom surface of the dielectric layer respectively, and are at least partially opposed to each other across the dielectric layer, the at least partially opposed portions of the first and the second electrode layers constitute a detection portion, and the sensor body is reversibly deformable to change areas of the top and bottom surfaces of the dielectric layer.

## Description

### TECHNICAL FIELD

The present invention relates to a swallowing movement measuring device, and a method for measuring a swallowing movement.

### BACKGROUND ART

In recent years, in medicinal and welfare fields, a patient's swallowing movement has been required to be measured in order to evaluate his/her swallowing function.

As a method for measuring a swallowing movement, for example, the following methods have been conventionally suggested as a noninvasive method: a method of measuring a patient's swallowing sound generated when he/she swallows; and a method of measuring a patient's myoelectricity that follows his/her muscly movement when he/she swallows.

However, these measuring methods have easily been affected by noises, so as to be desired to be improved.

Patent Literature 1 suggests a swallowing movement measuring device having a fitting portion to be fitted to an examinee to cover his/her cervical part; and a capacitive sensor set to this fitting portion, and contacts the surface of a peripheral area of either his/her thyroid cartilage or sternothyroid muscle when the fitting portion is fitted to the examinee.

### CITATION LIST

### PATENT LITERATURE

Patent Literature 1: Japanese Unexamined Patent Application Publication JP 2012-200300 A

### SUMMARY OF THE INVENTION

### TECHNICAL PROBLEM

In the swallowing movement measuring device disclosed in Patent Literature 1, as its capacitive sensor, a capacitive sensor that detects vibration (vibration capacitive sensor) is used.

When the vibration capacitive sensor is used to measure a vibration generated by an examinee's swallowing movement, and then the swallowing movement is measured on the basis of results of the measurement, the measurement results are easily affected by the vibration of the air around the examinee.

Moreover, according to the swallowing movement measuring device disclosed in Patent Literature 1, when its capacitive sensor is attempted to be brought into contact with an examinee's thyroid cartilage part, the capacitive sensor may not be easily fitted to the examinee since the thyroid cartilage part is sorely moved up and down (see Patent Literature 1, paragraph [0006]).

Furthermore, the invertors' investigations have made it evident that when a measurement is made in the state that a vibration capacitive sensor is brought into contact with an examinee's laryngeal prominence part of the body skin of his/her laryngeal part, the movement of his/her larynx is itself hindered.

Under such a situation, the inventors have investigated a new method for measuring a swallowing movement, and then accomplished the present invention.

### SOLUTION TO THE PROBLEM

The swallowing movement measuring device of the present invention is a device for measuring an examinee's swallowing movement on the basis of a movement of the examinee's larynx when the examinee swallows a bolus;
this device comprising a capacitive sensor to be brought into contact with a body skin of the examinee's laryngeal part, and a measuring unit that measures a change of the capacitive sensor in capacitance;
wherein the capacitive sensor comprises a sensor body comprising sheet-like dielectric layer; a first electrode layer and a second electrode layer,
the first and the second electrode layers are formed on a top surface and a bottom surface of the dielectric layer respectively, and are at least partially opposed to each other across the dielectric layer,

the at least partially opposed portions of the first and the second electrode layers constitute a detection portion, and
the sensor body is reversibly deformable to change areas of the top and bottom surfaces of the dielectric layer.

According to the swallowing movement measuring device of the present invention, the capacitive sensor, which has a soft sensor body that can be reversibly deformed in a surface direction thereof, is used to measure the movement of an examinee's larynx that is generated when the examinee swallows a bolus. Thus, without hindering a swallowing movement of the examinee, the swallowing movement is measurable.

In the capacitive sensor, a change in the capacitance depends on the deformation state of the sensor body in the surface direction. Thus, the capacitive sensor does not easily perceive any vibration as a noise source, so that the swallowing movement measuring device can precisely measure the examinee's laryngeal movement.

It is preferred in the swallowing movement measuring device that the dielectric layer comprises an elastomer composition.

The capacitive sensor having the dielectric layer, which comprises the elastomer composition, is particularly suitable for a sensor deformable in the surface direction in accordance with the examinee's laryngeal movement.

It is preferred in the swallowing movement measuring device that the sensor body has a plurality of the detecting portions.

In this case, a measurement of a change of each of the detecting portions in capacitance with time makes it possible to measure information pieces on the speed of an examinee's laryngeal movement generated when the examinee swallows a food bolus.

It is preferred that in the sensor body, the first electrode layer and the second electrode layer each comprise a plurality of rectangular electrodes arranged side by side to be separated from each other, and
the rectangular electrodes comprised in the first electrode layer and the rectangular electrodes comprised in the second electrode layer are arranged to be overlapped with each other in a thickness direction of the dielectric layer.

When the sensor body in the swallowing movement measuring device has the above-mentioned structure, the capacitive sensor is brought into contact with the body skin of the examinee's laryngeal part to render the direction in which the plural rectangular electrodes are arranged side by side a vertical direction and then a measurement is made. This manner makes it possible to measure, for example, the shift speed and the shift range of the examinee's laryngeal prominence part.

It is preferred in the swallowing movement measuring device that the capacitive sensor further comprises a fitting member; and
the fitting member is formed in such a manner that the capacitive sensor can be brought into contact with the body skin of the examinee's laryngeal part under a condition of rendering a state that the detecting portion or the detecting portions are stretched an initial state.

This case makes it possible to bring the capacitive sensor into contact with the body skin of the examinee's laryngeal part under a condition of rendering the state that the detecting portion of the sensor body is stretched the initial state. For this reason, a measurement can be made also about the examinee's laryngeal movement when the dielectric layer is contracted from the initial state.

The method for measuring the swallowing movement of the present invention is a method comprising:
bringing a capacitive sensor into contact with a body skin of an examinee's laryngeal part;
measuring a change of the capacitive sensor in capacitance, the change being based on a movement of the examinee's larynx when the examinee swallows a food bolus; and
measuring the examinee's swallowing movement on the basis of a result of the measurement;
wherein the capacitive sensor comprises a sensor body comprising sheet-like dielectric layer; a first electrode layer and a second electrode layer,
the first and the second electrode layers are formed on a top surface and a bottom surface of the dielectric layer respectively, and are at least partially opposed to each other across the dielectric layer,
the at least partially opposed portions of the first and the second electrode layers constitute a detection portion, and
the sensor body is reversibly deformable to change areas of the top and bottom surfaces of the dielectric layer.

In the method for measuring the swallowing movement of the present invention, the capacitive sensor, which has a soft sensor body that is reversibly deformable in a surface direction thereof, is used to measure the movement of the examinee's laryngeal part when the examinee swallows a food bolus. Thus, without hindering the examinee's swallowing movement, the method for measuring the swallowing movement makes it possible to measure the swallowing movement.

In the capacitive sensor, a change in the capacitance depends on the deformation state of the sensor body in the surface direction. Thus, the capacitive sensor does not easily perceive any vibration as a noise source, so that the method for measuring the swallowing movement makes it possible to measure the examinee's laryngeal part precisely, so that the examinee's swallowing movement can be precisely measured.

It is preferred in the method for measuring the swallowing movement that the dielectric layer comprises an elastomer composition.

The capacitive sensor having the dielectric layer, which comprises the elastomer composition, is particularly suitable for a sensor deformable in a surface direction thereof in accordance with the examinee's laryngeal movement.

It is preferred in the method for measuring the swallowing movement that the sensor body has a plurality of the detecting portions.

In this case, a measurement of a change of each of the detecting portions in capacitance with time makes it possible to measure information pieces on the speed of the examinee's laryngeal movement generated when the examinee swallows a food bolus.

It is preferred that in the sensor body, the first electrode layer and the second electrode layer each comprise a plurality of rectangular electrodes arranged side by side to be separated from each other, and
the rectangular electrodes comprised in the first electrode layer and the rectangular electrodes comprised in the second electrode layer are arranged to be overlapped with each other in a thickness direction of the dielectric layer.

When the sensor body in the method for measuring the swallowing movement has the above-mentioned structure, the capacitive sensor is brought into contact with the body skin of the examinee's laryngeal part to render the direction in which the plural rectangular electrodes are arranged side by side a vertical direction and then a measurement is made. This manner makes it possible to measure, for example, the shift speed and the shift range of the examinee's laryngeal prominence part.

It is preferred in the method for measuring the swallowing movement that the capacitive sensor is brought into contact with the body skin of the examinee's laryngeal part under a condition of rendering a state that the detecting portion or the detecting portions are stretched an initial state.

In this case, a measurement can be made also about the examinee's laryngeal movement when the dielectric layer is contracted from the initial state.

It is preferred in the method for measuring the swallowing movement that the capacitive sensor is brought into contact with of a laryngeal prominence part of the body skin of the laryngeal part.

In this case, the examinee's swallowing movement is more precisely measurable.

### ADVANTAGEOUS EFFECTS OF INVENTION

The present invention makes it possible to measure an examinee's swallowing movement without hindering the examinee's swallowing movement.

### BRIEF DESCRIPTION OF DRAWINGS

- FIG. 1: is a schematic view illustrating an example of a swallowing movement measuring device according to an embodiment of the present invention.
- FIG. 2: is a perspective view illustrating an example of a capacitive sensor which the swallowing movement measuring device according to the embodiment of the present invention has.
- FIG. 3A: is a perspective view illustrating an example of a sensor body included in the capacitive sensor illustrated in FIG. 2, and
- FIG. 3B: is a line A-A sectional view of FIG. 3A.
- FIG. 4A and FIG. 4B: are each a schematic view illustrating a state that the capacitive sensor illustrated in FIG. 2 is fitted to an examinee.
- FIG. 5: is a perspective view illustrating another example of the capacitive sensor which the swallowing movement measuring device according to the embodiment of the present invention has.
- FIG. 6A and FIG. 6B: are views illustrating still another example of the capacitive sensor which the swallowing movement measuring device according to the embodiment of the present invention has; and FIG. 6A is a plan view thereof, and FIG. 6B is a rear surface view thereof.
- FIG. 7: is a plan view illustrating another example of the capacitive sensor which the swallowing movement measuring device according to the embodiment of the present invention has.
- FIG. 8A: is a perspective view illustrating an example of a sensor body included in the capacitive sensor in FIG. 7, and
- FIG. 8B: is an exploded perspective view of FIG. 8A.
- FIG. 9A and FIG. 9B: are each a schematic view illustrating a state that the capacitive sensor illustrated in FIG. 7 is fitted to an examinee.
- FIG. 10: is a schematic view of a forming apparatus used in the Example.
- FIG. 11A to FIG. 11D: are perspective views referred to for describing a process for producing a sensor body A in the Example.
- FIG. 12A and FIG. 12B: are each a view referred to for describing an examinee's position where a capacitive sensor in Example 1 is fitted to the examinee.
- FIG. 13: is a view of graphs showing a capacitance change measured in Example 1.
- FIG. 14A and FIG. 14B: are graphs showing a capacitance change measured in Example 2.
- FIG. 15: is a view of graphs showing a capacitance change measured in Example 3.
- FIG. 16: is a graph showing a capacitance change measured in Example 4.

### DESCRIPTION OF EMBODIMENTS

Hereinafter, embodiments of the present invention will be described with reference to the drawings.

### First Embodiment

A swallowing movement measuring device according to the present embodiment is a device for measuring an examinee's swallowing movement on the basis of a movement of the examinee's larynx when the examinee swallows a food bolus, and this device includes a capacitive sensor to be brought into contact with a body skin of the examinee's laryngeal part, and a measuring unit that measures a change of the capacitive sensor in capacitance.

A method for measuring a swallowing movement according to the present embodiment is a method comprising: bringing a capacitive sensor into contact with a body skin of an examinee's laryngeal part, measuring a change of the capacitive sensor in capacitance, this change being based on a movement of the examinee's larynx when the examinee swallows a food bolus, and measuring the examinee's swallowing movement on the basis of a result of the measurement.

In the present embodiment, the capacitive sensor includes a sensor body that has a sheet-like dielectric layer made of an elastomer composition, and a first electrode layer and a second electrode layer that are each made of an electro-conductive composition including an electro-conductive material. The first and the second electrode layers are formed on a top surface and a bottom surface of the dielectric layer respectively, and are at least partially opposed to each other across the dielectric layer. The at least partially opposed portions of the first and the second electrode layers constitute a detection portion, and the sensor body is reversibly deformable to change areas of the top and bottom surfaces of the dielectric layer.

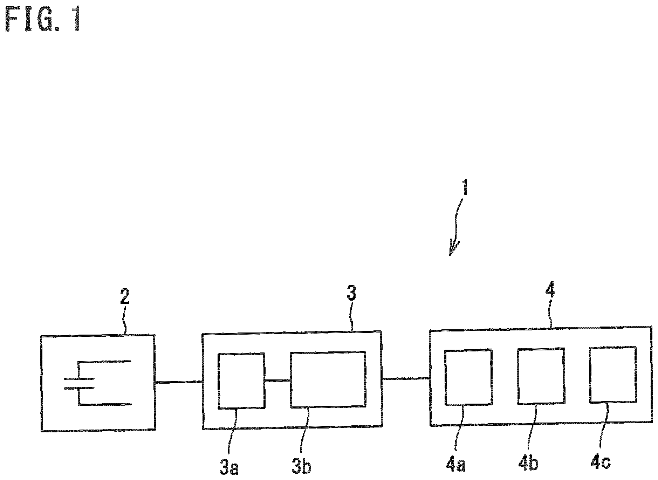

FIG. 1 is a schematic view illustrating an example of a swallowing movement measuring device according to the present embodiment.

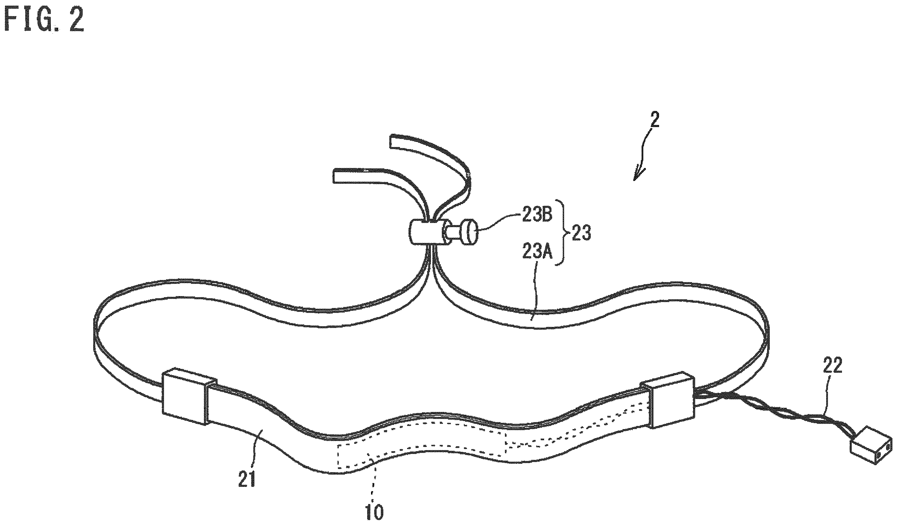

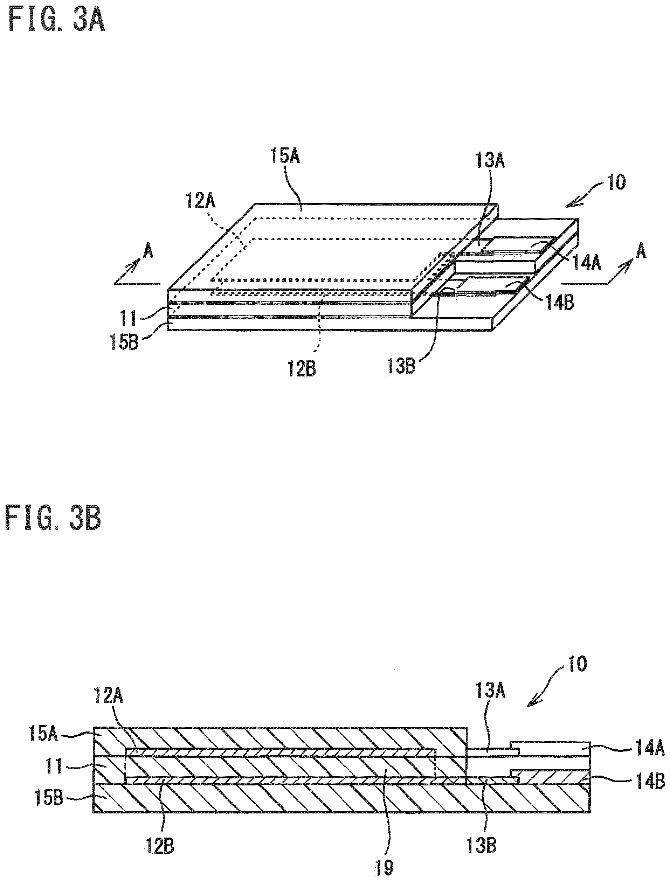

FIG. 2 is a perspective view illustrating an example of a capacitive sensor which the swallowing movement measuring device according to the present embodiment has. FIG. 3A is a perspective view illustrating a sensor body included in the capacitive sensor illustrated in FIG. 2. FIG. 3B is a line A-A sectional view of FIG. 3A.

As illustrated in FIG. 1, a swallowing movement measuring device 1 according to the present embodiment has the capacitive sensor, which is a capacitive sensor 2, a measuring unit 3 connected electrically to the capacitive sensor 2, and an indicator 4 that indicates measured results in the measuring unit 3.

The measuring unit 3 is equipped with a Schmitt trigger oscillation circuit 3a for converting a capacitance C to a frequency signal F, an F/V conversion circuit 3b for converting the frequency signal F to a voltage signal V, and a power supply circuit (not illustrated). The measuring unit 3 converts the capacitance C of a detecting portion of the capacitive sensor 2 to the frequency signal F, and subsequently makes a further conversion thereof to the voltage signal V to transmit this signal to the indicator 4.

The indicator 4 has a monitor 4a, an arithmetic circuit 4b, and a memory 4c. The indicator 4 causes a change in the capacitance C measured by the measuring unit 3 to be indicated on the monitor 4a. Moreover, in the indicator 4, the change in the capacitance C is memorized as recorded data in the memory 4c.

As illustrated in FIG. 2, the capacitive sensor 2 has a sensor body 10, a covering member 21 located around the sensor body 10, a connecting member 22 for connecting the sensor body 10 electrically to the measuring unit 3, and a fitting member 23 for fitting the capacitive sensor 2 to an examinee.

As illustrated in FIGS. 3A and 3B, the sensor body 10 has a sheet-like dielectric layer 11 made of an elastomer composition; a top electrode layer 12A formed on the top surface of the dielectric layer 11; a bottom electrode layer 12B formed on the bottom surface of the dielectric layer 11; a top conductive wire 13A linked to the top electrode layer 12A; a bottom conductive wire 13B linked to the bottom electrode layer 12B; a top connecting portion 14A fitted to an end of the top conductive wire 13A that is opposite to the top electrode layer 12A; a bottom connecting portion 14B fitted to an end of the bottom conductive wire 13B that is opposite to the bottom electrode layer 12B; and a top protective layer 15A and a bottom protective layer 15B laminated, respectively, onto the top side and the bottom side of the dielectric layer 11.

The top electrode layer 12A and the bottom electrode layer 12B have the same shape when viewed in plan. The whole of the top electrode layer 12A, and that of the bottom electrode layer 12B are opposed to each other to sandwich the dielectric layer 11 therebetween. In the capacitive sensor 2, its portion where the top electrode layer 12A and the bottom electrode layer 12B are opposed to each other is the detecting portion 19. The sensor body 10 has a single detecting portion, that is, the detecting portion 19.

In the sensor body 10, the top electrode layer 12A corresponds to a first electrode layer; and the bottom electrode layer 12B corresponds to a second electrode.

In the sensor body 10, the dielectric layer 11 is made of an elastomer composition. Thus, the sensor body 10 can be deformed (stretched and contracted) in a surface direction thereof. At the time of the deformation of the dielectric layer 11 in the surface direction in the sensor body 10, following the deformation of the dielectric layer the following layers are deformed: the top electrode layer 12A and the bottom electrode layer 12B; and the top protective layer 15A and the bottom protective layer 15B (hereinafter, a combination of these two may be referred to as the protective layers).

About the capacitive sensor 2, therefore, following a deformation of the sensor body 10, the capacitance of the detecting portion 19 is changed correlatively (proportionally) with the deformation quantity of the dielectric layer 11. For this reason, when a change of the detecting portion 19 in capacitance is detected, the deformation quantity of the sensor body 10 can be detected.

Returning to FIG. 2, the covering member 21 that is made of stretchable cloth is laid to both surfaces of the sensor body 10. The covering member 21 comprises two stretchable cloth pieces which are each larger in longitudinal direction length than the sensor body 10. The sensor body 10 is sandwiched between the two cloth pieces. By the laminating of the covering member 21, the sensor body 10 can be protected.

The fitting member 23 is fitted to both ends of the sensor body 10 (the covering member 21).

The fitting member 23 is a member for fitting the capacitive sensor 2 to an examinee. The fitting member 23 has stretchable moieties 23A that are, for example, rubbery strings fitted, respectively, to both ends of the covering member 21, and an adjustor 23B for adjusting the length of the stretchable moieties 23A when the capacitive sensor 2 is fitted to the examinee.

The following will describe a use method of the swallowing movement measuring device 1.

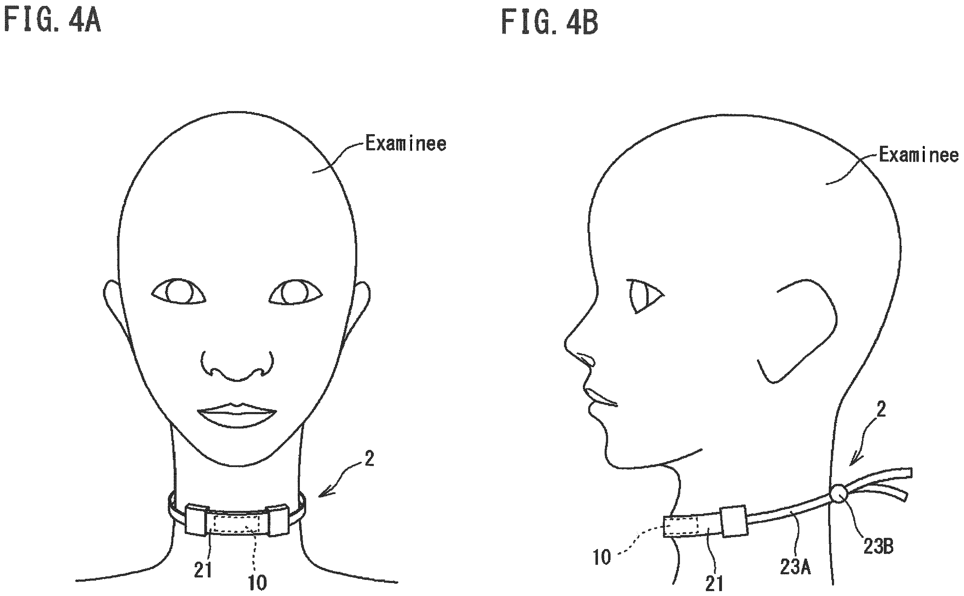

FIGS. 4A and 4B are each a schematic view illustrating a state that the capacitive sensor 2 is fitted to an examinee.

The swallowing movement measuring device 1 is used in the state of being brought into contact with the body surface of the examinee's laryngeal part.

As illustrated in FIGS. 4A and 4B, the capacitive sensor 2 is fitted to the examinee in the manner that a portion of the covering member 21 that covers the detecting portion 19 of the sensor body 10 contacts the body surface of the examinee's laryngeal part. At this time, on the backside of the examinee's neck, the length of the stretchable moieties 23A is adjusted with the adjustor 23B, so that the covering member 21 can maintain the state of contacting the body surface of the laryngeal part.

In the swallowing movement measuring device 1, the capacitive sensor 2 is fitted to the examinee as described above. In this state, this device measures the movement of the examinee's larynx.

Specifically, the examinee is caused to swallow a food bolus of, for example, a solid, semisolid or liquid. The movement of the examinee's larynx that is generated at this time is measured. When the examinee swallows the food bolus, the larynx is moved. Following this movement, the sensor body 10 (the dielectric layer 11) is deformed in a surface direction thereof.

As a result, the detecting portion 19 is changed in capacitance in accordance with the deformation quantity of the sensor body 10 (the dielectric layer 11). Accordingly, when the measuring unit 3 measures the capacitance of the detecting portion 19, and the resultant change in the capacitance is monitored in the indicator 4, the movement state of the larynx can be comprehended, this state being based on swallowing action of the examinee.

In the present embodiment, the examinee's site with which the capacitive sensor 2 is brought into contact is not particularly limited as far as the site is in the body surface of the examinee's larynx. The site is preferably a site where the sensor contacts the examinee's laryngeal prominence part.

The laryngeal prominence part is a region that is largely moved up and down when the examinee swallows a food bolus. The examinee's site where the sensor contacts the examinee's laryngeal prominence part, the state of this region being easily understood by touching the body surface, is suitable for measuring the examinee's swallowing movement.

When the capacitive sensor 2 is brought into contact with the body surface of the laryngeal part in the present embodiment, it is preferred that the capacitive sensor 2 is brought into contact with the body surface of the laryngeal part under a condition of rendering not a non-stretched state of the dielectric layer 11 of the sensor body 10 but a slightly stretched state thereof an initial state. In this case, the movement of the larynx is measurable also when the larynx is moved to cause the dielectric layer 11 from the initial state to contract.

In order to render the state that the dielectric layer 11 is slightly stretched the initial state, it is advisable to bring the capacitive sensor 2 into contact with the body surface, for example, to cover at least one portion of the laryngeal prominence part with the sensor body 10.

In the present embodiment, the shape and the size of the detecting portion 19 of the sensor body 10 are not particularly limited. It is preferred that the shape is a rectangular shape when viewed in plan, and the length of each of its long sides is from 10 to 100 mm, and that of each of its short sides is from 5 mm to 30 mm.

A case where the sensor body 10 has the detecting portion 19 having such a size is more suitable for a swallowing movement measurement in the state that the sensor body 10 is brought into contact with the examinee in the state of covering at least one portion of the laryngeal prominence part.

### Second Embodiment

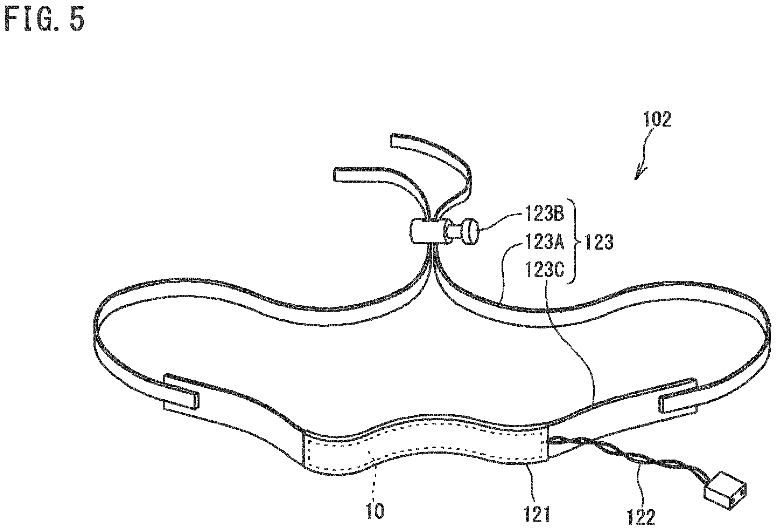

In an embodiment of the present invention, the above-mentioned capacitive sensor is not limited to that illustrated in FIG. 2, and may be, for example, a capacitive sensor as illustrated in FIG. 5.

FIG. 5 is a perspective view illustrating an example of a capacitive sensor which a swallowing movement measuring device according to the present embodiment has.

As illustrated in FIG. 5, a capacitive sensor 102 has a sensor body 10; a covering member 121 located around the sensor body 10; a connecting member 122 for connecting the sensor body 10 electrically to a measuring unit; and a fitting member 123 for fitting the capacitive sensor 102 to an examinee. The sensor body 10 which the capacitive sensor 102 has is equivalent to the sensor body which the capacitive sensor 2 has.

The covering member 121 that is made of silicone elastomer is formed around the sensor body 10.

The formation of the covering member 121 makes it possible to protect the sensor body 10. The covering member 121 made of silicone elastomer is excellent, particularly, in water resistance; thus, this swallowing movement measuring device is suitable for protecting the sensor body 10, for example, when the examinee sweats.

A fitting member 123 is located to both ends of the sensor body 10 (the covering member 121).

The fitting member 123 is a member for fitting the sensor body 102 to an examinee. The fitting member 123 has linking moieties 123C each made of, for example, a cloth tape and fitted, respectively, to ends of the sensor body 10 (the covering member 121), stretchable moieties 123A that are, for example, rubbery strings fitted, respectively, to both ends of the linking moieties 123C that are opposite to the sensor body 10; and an adjustor 123B for adjusting the length of the stretchable moieties 123A when the capacitive sensor 102 is fitted to the examinee.

The linking moieties 123C are each a member that is sufficiently smaller in stretchability than the stretchable moieties 123A (preferably a member that is not substantially stretched nor contracted). This matter makes it possible to avoid the following when the sensor body 10 is stretched or contracted (deformed) by a movement of the examinee's larynx: the fitting member 123 is stretched or contracted in accompaniment with the movement of the examinee's larynx. As a result, the deformation quantity of the sensor body 10 is increased, so that the swallowing movement measuring device having the sensor body 102 can measure the movement of the larynx in more detail.

The capacitive sensor 102 can also be fitted to the examinee in the same way as the capacitive sensor 2.

### Third Embodiment

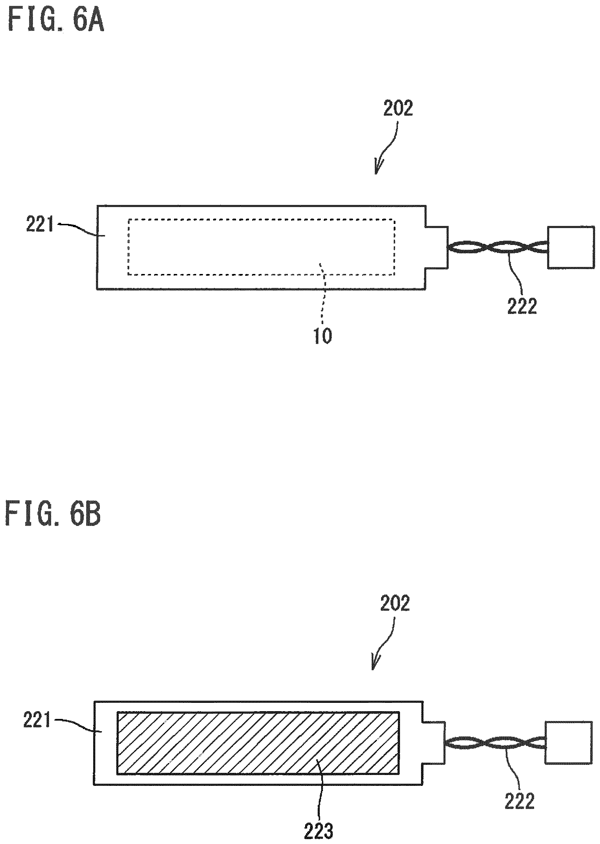

In an embodiment of the present invention, the above-mentioned capacitive sensor may be, for example, a capacitive sensor as illustrated in FIG. 6A and FIG. 6B.

FIG. 6A and FIG. 6B are views illustrating an example of a capacitive sensor which the swallowing movement measuring device according to the present embodiment has. FIG. 6A is a plan view thereof, and FIG. 6B is a rear surface view thereof.

As illustrated in FIGS. 6A and 6B, a capacitive sensor 202 has a sensor body 10; a covering member 221 located around the sensor body 10; a connecting member 222 for connecting the sensor body 10 electrically to a measuring unit; and an adhesive layer 223 for causing the capacitive sensor 202 to adhere to an examinee. The sensor body 10 which the capacitive sensor 202 has is equivalent to the sensor body which the capacitive sensor 2 has.

The covering member 221 that is made of stretchable cloth is laid on both surfaces of the sensor body 10.

The covering member 221 comprises two stretchable cloth pieces which are each slightly larger in size than the sensor body 10 when viewed in plan. The sensor body 10 is sandwiched between these two cloth pieces.

By the laminating of the covering member 221, the sensor body 10 can be protected.

The adhesive layer 223 is laid on one of the two surfaces of the covering member 221.

The adhesive layer 223 is a member for fitting the capacitive sensor 202 to an examinee.

The capacitive sensor 202 is caused to adhere onto the body skin of the examinee's laryngeal part through the adhesive layer 223 to be fittable to the examinee.

In the capacitive sensor 202, the adhesive layer 223 functions as a fitting member.

The capacitive sensors 2 and 102 are each a sensor fitted to the examinee into a lateral direction (in a direction along a direction around the examinee's neck). In contrast, the capacitive sensor 202 having the adhesive layer 223 is fittable to the examinee not only in a lateral direction but also in a vertical direction or any other direction.

### Fourth Embodiment

In each of the first to the third embodiments, the sensor body 10 is a sensor body having a single detecting portion. However, the swallowing movement measuring device according to an embodiment of the present invention may have plural detecting portions.

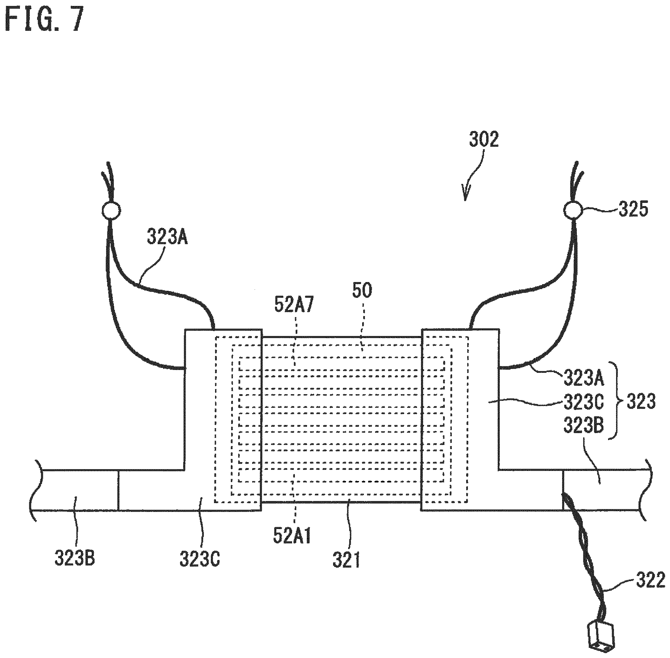

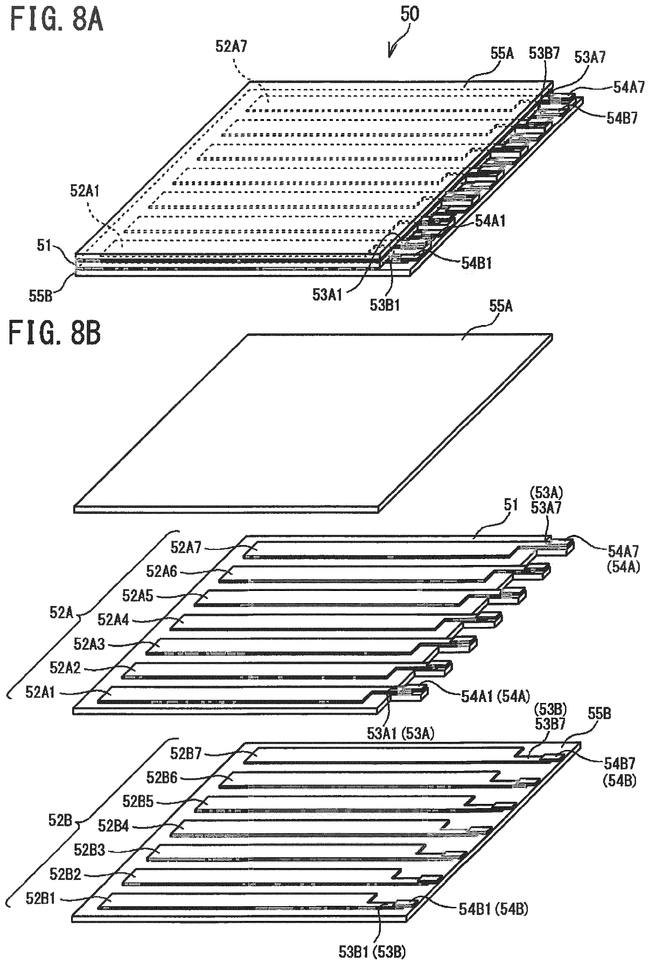

FIG. 7 is a plan view illustrating an example of a capacitive sensor which the swallowing movement measuring device according to the present embodiment has. FIG. 8A is a perspective view illustrating an example of a sensor body included in the capacitive sensor illustrated in FIG. 7. FIG. 8B is an exploded perspective view of FIG. 8A.

A capacitive sensor 302 is a capacitive sensor having a sensor body 50 having plural detecting portions. As illustrated in FIG. 7, the capacitive sensor 302 has the sensor body 50; a covering member 321 located around the sensor body 50; a connecting member 322 for connecting the sensor body 50 electrically to a measuring unit; and a fitting member 323 for fitting the capacitive sensor 302 to an examinee.

As illustrated in FIG. 8A, the sensor body 50 has a sheet-like dielectric layer 51 made of an elastomer composition; and a top electrode layer 52A formed on the top surface of the dielectric layer 51, and top conductive wires 53A linked to this top electrode layer 52A; a bottom electrode layer 52B formed on the bottom surface of the dielectric layer 51, and bottom conductive wires 53B linked to the bottom electrode layer 52B.

As illustrated in FIG. 8B, the top electrode layer (first electrode layer) 52A comprises seven rectangular electrodes 52A1 to 52A7 arranged apart from each other to be at regular intervals side by side. The top conductive wires 53A (53A1 to 53A7) are each smaller in width than each of the rectangular electrodes, and they are linked to the seven rectangular electrodes 52A1 to 52A7, respectively.

As illustrated in FIG. 8B, the bottom electrode layer (second electrode layer) 52B comprises seven rectangular electrodes 52B1 to 52B7 arranged apart from each other to be at regular intervals side by side. The bottom conductive wires 53B (53B1 to 53B7) are each smaller in width than each of the rectangular electrodes, and they are linked to the seven rectangular electrodes 52B1 to 52B7, respectively.

The rectangular electrodes 52A1 to 52A7, which constitute the first electrode layer 52A, and the rectangular electrodes 52B1 to 52B7, which constitute the second electrode layer 52B, are arranged side by side in the same direction (vertical direction in FIG. 7) out of surface directions of the dielectric layer 51. The rectangular electrodes 52A1 to 52A7, and the rectangular electrodes 52B1 to 52B7 are disposed at positions of the sensor where the former electrodes 52A1 to 52A7 are overlapped with the latter electrodes 52B1 to 52B7, respectively, in the thickness direction of the dielectric layer 51.

In other words, the top electrode layer 52A and the bottom electrode layer 52B have the same shape when viewed in plan. The whole of the top electrode layer 52A and that of the bottom electrode layer 52B are opposed to each other to sandwich the dielectric layer 51 therebetween. In the capacitive sensor 302, its portions where the following is satisfied are the detecting portions: the rectangular electrodes 52A1 to 52A7 are opposed to the rectangular electrodes 52B1 to 52B7 (its portion where the rectangular electrode 52A1 is opposed to the rectangular electrode 52B1, its portion where the rectangular electrode 52A2 is opposed to the rectangular electrode 52B2, and the like). The sensor body 50 has seven detecting portions, that is, the detecting portions referred to herein.

Moreover, the sensor body 50 has top connecting portions 54A1 to 54A7 fitted to respective ends of the top conductive wires 53A1 to 53A7 that are opposite to the top electrode layer 52A, and bottom connecting portions 54B1 to 54B7 fitted to respective ends of the bottom conductive wires 53B1 to 53B7 that are opposite to the bottom electrode layer 52B.

The sensor body 50 further has a top protective layer 55A and a bottom protective layer 55B, respectively, on the top side and the bottom side of the dielectric layer 51.

In the sensor body 50, the dielectric layer 51 is made of an elastomer composition. Thus, in the same way as the sensor body 10, the sensor body 50 can be deformed (stretched and contracted) in a surface direction thereof. At the time of the deformation of the dielectric layer 51 in the surface direction in the sensor body 50, following this deformation, the top electrode layer 52A and the bottom electrode layer 52B, and the protective layers 55A and 55B are deformed.

About the capacitive sensor having the sensor body 50, therefore, following a deformation of the sensor body 50, the capacitance of each of the detecting portions is changed correlatively with the deformation quantity of the dielectric layer 51.

Returning to FIG. 7, the covering member 321 that is made of stretchable cloth is laid on the periphery of the sensor body 50. The covering member 321 comprises stretchable cloth pieces which are each slightly larger in size than the sensor body 50 when viewed in plan. The sensor body 50 is sandwiched between these cloth pieces, the number of which is two. By the laminating of the covering member 321, the sensor body 50 can be protected.

The fitting member 323 is fitted to both ends of the sensor body 50 (outside both ends in the longitudinal direction of each of the rectangular electrodes).

The fitting member 323 is a member for fitting the capacitive sensor 302 to an examinee. The fitting member 323 has linking moieties 323C fitted, respectively, to both ends of the sensor body 50 (covering member 321) and made of non-stretchable cloth; loop-form first fitting moieties 323A fitted, respectively, to the linking moieties 323C and each made of, for example, a rubbery string; and second fitting moieties 323B fitted to the linking moieties 323C and made of, for example, hook and loop fastener parts.

In substantially the same way as the capacitive sensor 2, the capacitive sensor 302 can be fitted to an examinee.

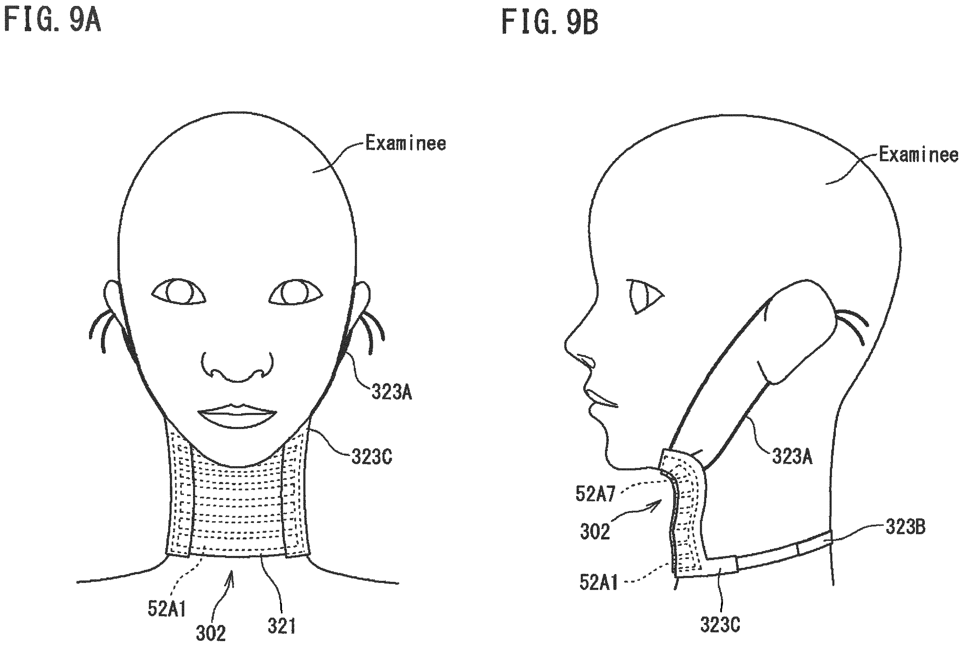

FIGS. 9A and 9B are each a schematic view illustrating a state that the capacitive sensor 302 is fitted to the examinee.

As illustrated in FIGS. 9A and 9B, while a portion of the covering member 321 that covers the sensor body 50 is brought into contact with the body surface of the examinee's laryngeal part, the capacitive sensor 302 is fitted to the examinee to cover the body surface of the laryngeal part.

Specifically, while the respective lengths of the first fitting moieties 323A are adjusted with the adjustors 325, the first fitting moieties 323A are hung over the examinee's ears. Simultaneously, the second fitting moieties 323B are wound around the examinee's neck, and then fixed to each other through the hook and loop fastener, so that the capacitive sensor 302 can be fitted to the examinee at the examinee's predetermined position.

In the swallowing movement measuring device having the capacitive sensor 302, the capacitive sensor 302 is fitted to the examinee as described above. In this state, the movement of the examinee's larynx is measured.

The capacitive sensor 302 is fitted to the examinee to make the direction along which a plurality of the detecting portions are arranged side by side consistent with the shifting direction (vertical direction) of the examinee's laryngeal prominence part.

Accordingly, when the respective capacitances of the individual detecting portions are measured over a predetermined period, the shifting speed and the shifting range of the laryngeal prominence part can be measured on the basis of a change of each of the detecting portions in capacitance.

In short, the use of the swallowing movement measuring device according to the present embodiment makes it possible to measure information pieces on the movement of the larynx in more detail when the examinee swallows.

### Other Embodiments

In the present embodiment, the swallowing movement measuring device may have plural capacitive sensors each having one detecting portion. In this case, the plural capacitive sensors are simultaneously brought into contact with the body skin of an examinee's larynx. The plural capacitive sensors are fitted thereto, for example, along the sift direction (vertical direction) of the examinee's laryngeal prominence part. The fitting makes it possible to measure, in more detail, the shift speed and the sift range of the examinee's laryngeal prominence part, and other information pieces on the examinee's swallowing movement.

The swallowing movement measuring device may have plural capacitive sensors each having plural detecting portions.

When a single sensor body has plural detecting portions in any embodiment of the present invention, individual detecting portions may not be necessarily arranged at regular intervals in one direction as attained in the fourth embodiment. As far as an initial-state positional relationship between the individual detecting portions can be grasped, measurements can be made about, for example, the shift speed and the sift range of an examinee's laryngeal part in the examinee's swallowing movement.

In each of the embodiments of the present invention, it is not necessarily essential that the whole of the top electrode layer is opposite to that of the bottom electrode layer, these layers being possessed by the sensor body, to sandwich the dielectric layer therebetween. Thus, is sufficient for these layers to be at least partially opposite to each other.

The following will describe, in detail, each of the members which the swallowing movement measuring device according to each of the embodiments of the present invention has.

### Capacitive Sensor

### Dielectric Layer

The dielectric layer is a sheet-like member made of an elastomer composition. The dielectric layer can be reversibly deformed to change the top and bottom surfaces thereof in area. In the present invention, the top and bottom surfaces of the sheet-like dielectric layer mean the top surface and the bottom surface of the dielectric layer.

An example of the elastomer composition is a composition which includes an elastomer and includes, as required, any other optional component. The elastomer is preferably a urethane elastomer or a silicone elastomer since the elastomers are small in permanent strain (or permanent elongation).

The average thickness of the dielectric layer is preferably from 10 to 1000 µm to make this layer large in capacitance C to improve the measuring device in detecting sensitivity, and to make the measuring device better in performance of following a measuring object.

It is preferred that the dielectric layer can be deformed to increase the area of the top and bottom surfaces thereof by 30 % or more from a non-elongated state of the layer. When the dielectric layer, which has such a characteristic, is used in the state of being bonded to the body skin of an examinee's laryngeal part, the dielectric layer is suitable for following a deformation of the body skin of the laryngeal part, so as to be deformed.

Such a wording "the dielectric layer can be deformed to increase the area by 30 % or more" means that even when a load is applied onto the dielectric layer to increase the area by 30 %, this layer is not broken, and further when the load is cancelled, the layer is restored to an original state thereof (in other words, the layer is deformed within an elastic deformation range thereof).

The range of the dielectric layer that can be deformed in surface directions thereof is controllable by designing (the material, the shape or some other factor of) the dielectric layer.

### Electrode Layers (Top Electrode Layer and Bottom Electrode Layer)

The electrode layers are each made of an electroconductive composition including an electroconductive material.

The top electrode layer and the bottom electrode layer are usually formed by using the same material. However, it is not necessarily essential to use the same material.

Examples of the electroconductive material include carbon nanotubes, electroconductive carbon black, graphite, metallic nanowires, metallic nanoparticles, and an electroconductive polymer. These may be used singly or in any combination of two or more thereof.

The electroconductive composition may include, besides the electroconductive material, a binder component functioning as a binding material for the electroconductive material, and various additives. Examples of the additives include a dispersing agent for the electroconductive material, a crosslinking agent for the binder component, a vulcanization promoter, a vulcanization aid, an antioxidant, a plasticizer, a softener, and a colorant.

### Others

As attained in the examples illustrated in FIGS. 2 and 8, at the need arises, a sensor body as described above may optionally have a first conductive wire (top conductive wire) and a second conductive wire (bottom conductive wire) connected to electrode layers, respectively.

These conductive wires are sufficient to be conductive wires which do not hinder any deformation of the dielectric layer, and maintain electro-conductivity even when the dielectric layer is deformed. A specific example of the conductive wires is conductive wires made of the same electro-conductivity composition as used for the electrode layers.

As attained in the examples in illustrated FIGS. 2 and 8, at the need arises, at an end of each of the conductive wires, the end being opposite to the electrode layer side of the conductive wire, a top connecting portion or a bottom connecting portion may be located for connecting an external conductive wire. Each of these connecting moieties is, for example, a connecting portion made of copper foil.

As seen in the examples in illustrated FIGS. 2 and 8, in the sensor body, one or more protective layers may be laminated onto the outermost layer on the top side of the sensor body, and/or the outermost layer on the bottom side thereof. The laminating of the protective layer(s) makes it possible to enhance the sensor body in strength and endurance.

The material of the protective layer(s) is not particularly limited. It is advisable to select the material appropriately in accordance with required properties thereof. A specific example thereof is an elastomer material equivalent to the material of the dielectric layer.

### Measuring Unit

The measuring unit is connected electrically to the capacitive sensor. The measuring unit has a function of measuring the capacitance C of the detecting portion, or each of the detecting portions that is changed in accordance with the deformation of the dielectric layer. The method for measuring the capacitance C may be a method known in the prior art. Accordingly, the measuring unit has, for example, a capacitance measuring circuit, an arithmetic circuit, an amplifier circuit, and a power supply circuit that are required.

Examples of a method (or circuit) for measuring the capacitance C include a CV conversion circuit using an automatic balanced bridge circuit (such as an LCR meter), a CV conversion circuit using an inverting amplifier circuit, a CV conversion circuit using a half-wave voltage double rectification circuit, a CF oscillation circuit using a Schmitt trigger oscillation circuit, and a method using a combination of a Schmitt trigger oscillation circuit with a F/V conversion circuit.

### Indicator

As has been illustrated in FIG. 1, the swallowing movement measuring device according to each of the embodiments of the present invention may have an indicator. This matter makes it possible that a user of the swallowing movement measuring device checks a change of the capacitance C (and information pieces on the swallowing movement that are based on this change) at a real time. The indicator has, for example, a monitor, an arithmetic circuit, an amplifier circuit and a power supply circuit that are required for the check.

The indicator may have a memory, such as a RAM, a ROM or an HDD, for memorizing measured results of the capacitance C.

The measuring unit may have this memory.

As the indicator, a terminal unit may be used, such as a personal computer, a smart phone, or a tablet terminator.

In the swallowing movement measuring device 1 illustrated in FIG. 1, the measuring unit 3 is connected to the indicator 4 through a wire; however, the two may be wirelessly connected to each other.

### EXAMPLES

Hereinafter, the present invention will be specifically described by way of Examples thereof. However, embodiments of the present invention are not limited to the Examples.

### Production of Sensor Body A

A sensor body was produced which had substantially the same structure as the sensor body 10 in FIG. 3.

### (1) Production of Dielectric Layer

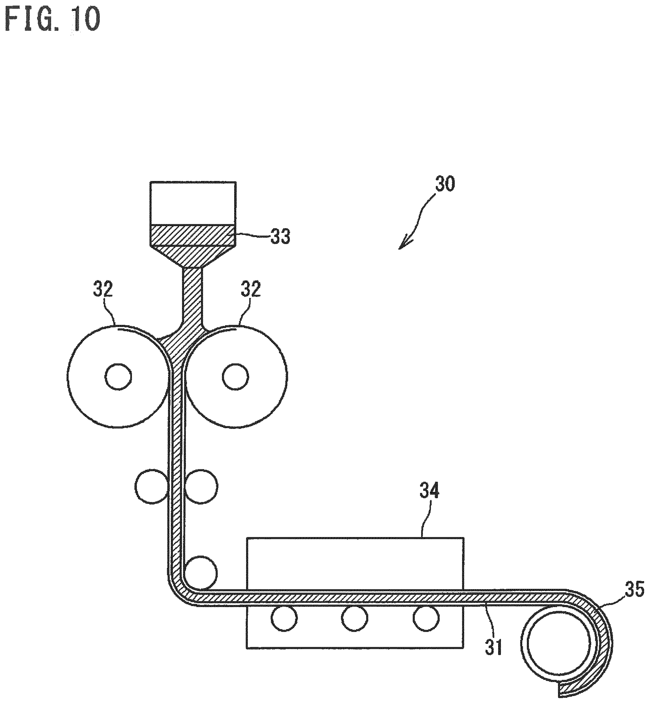

To 100 parts by mass of a polyol (PANDEX GCB-41, manufactured by DIC Corp.) were added 40 parts by mass of a plasticizer (dioctyl sulfonate) and 17.62 parts by mass of an isocyanate (PANDEX GCA-11, manufactured by DIC Corp.). An agitator was used to agitate and mix these components with each other to prepare a starting composition for dielectric layer. Next, the starting composition 33 was poured into a forming apparatus 30 illustrated in FIG. 10. While protective films 31 were used to make the composition 33 into a sandwich form and further the sandwich is transported, the composition 33 was crosslinked and cured under the following conditions.

An inside temperature of furnace 34 was 70 °C and a furnace-inside period was 30 minutes. In this way, a rolled sheet 35 was yielded which had the protective films and a predetermined thickness. The composition of the sheet was then crosslinked in a furnace having a temperature adjusted to 70 °C after 12 hours to yield a sheet made of a polyether-based urethane elastomer. The resultant urethane sheet was cut into each piece having a size of 14 mm x 70 mm x 100 µm thickness. Furthermore, one corner of the piece was cut into a size of 7 mm x 7 mm x 100 µm thickness. In this way, each dielectric layer was produced.

About the produced dielectric layers, the elongation (%) at breaking and the dielectric constant thereof were measured. The elongation (%) at breaking was 505 %, and the dielectric constant was 5.8.

The elongation at breaking was measured according to JIS K 6251. The dielectric constant was obtained by sandwiching one of the dielectric layers between electrodes of 20 mm diameter, using an LCR HITESTER (3522-50, manufactured by Hioki E. E. Corp.) to measure the capacitance at a measuring frequency of 1 kHz, and then making a calculation from the electrode area and the thickness of the measured sample.

### (2) Preparation of Electrode Layer Material

To 30 g of methyl isobutyl ketone (MIBK) was added 30 mg of highly-oriented carbon nanotubes manufactured by Taiyo Nippon Sanso Corp. Ajet mill (NANOJET PAL JN10-SP003, manufactured by Jokoh Co., Ltd.) was used to subject the resultant to a wet dispersing treatment. The resultant liquid was diluted 10 times to yield a carbon nanotube dispersed liquid having a concentration of 0.01 % by weight.

### (3) Production of Protective Layers

The following protective layers were produced, using the same method as in the above-mentioned item (1) Production of Dielectric Layer except that the sheet thickness was changed: a bottom protective layer made of a polyether-based urethane elastomer and having a size of 14 mm x 70 mm x 50 µm thickness; and a top protective layer made of the same elastomer and having a size of 14 mm x 63 mm x 50 µm thickness.

### (4) Production of Sensor Body

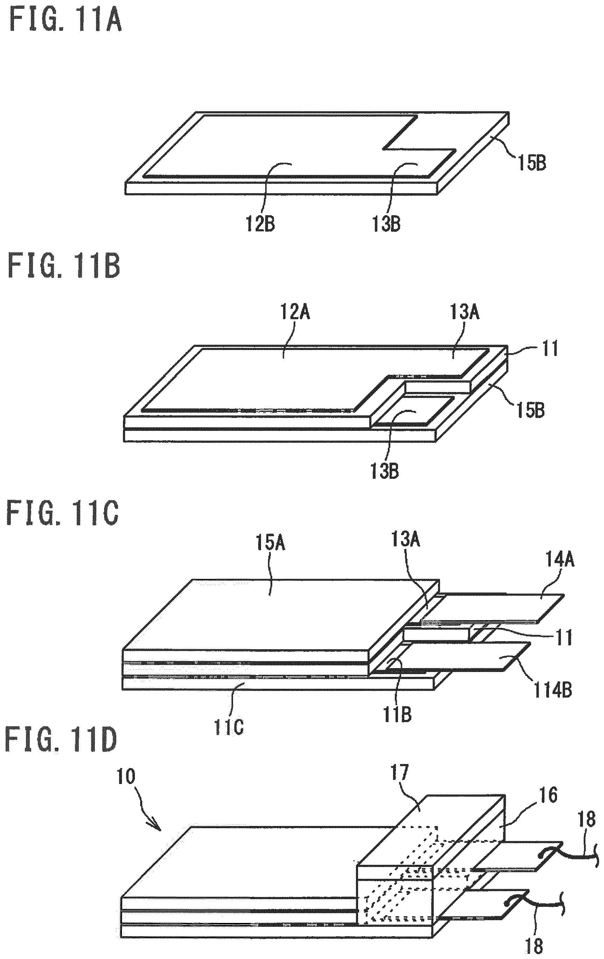

FIG. 11A to FIG. 11D are each a perspective view referred to for describing a producing process of a sensor body A in the Example.

Initially, a mask (not illustrated) was caused to adhere onto a single surface (top surface) of the bottom protective layer 15B produced in the step of the item (3). The mask was a mask in which openings each having a predetermined shape were made in a PET film subjected to release-treatment.

In the mask, an opening corresponding to the position of the bottom electrode layer and the bottom conducting wire is formed. The size of the opening is such that the portion corresponding to the bottom electrode layer has a size of 10 mm in width × 60 mm in length, and the portion corresponding to the bottom conducting wire has a size of 5 mm in width × 6 mm in length.

Next, an air blush was used to paint 7.2 g of the carbon nanotube dispersed liquid prepared in the step of the item (2) onto the workpiece. At this time, the distance between an injection tip of the air brush and the liquid-painted surface of the bottom protective layer 15B was set to 10 cm. Subsequently, the workpiece was dried at 100 °C for 10 minutes to form a bottom electrode layer 12B and a bottom conductive wire 13B. Thereafter, the mask was peeled away (see FIG. 11A).

Next, a dielectric layer 11 was laminated onto the bottom protective layer 15B. In this case, the dielectric layer 11, which was one of the dielectric layers 11 produced in the step of the above-mentioned item (1), was caused to adhere onto the bottom protective layer 15B to cover the whole of the bottom electrode layer 12B and one portion of the bottom conductive wire 13B.

Furthermore, a top electrode layer 12A and a top conductive wire 13A were formed (see FIG. 11B) on the top side of the dielectric layer 11. The formation of the top electrode layer 12A and the top conductive wire 13A was attained in the same manner as used to form the bottom electrode layer 12B and the bottom conductive wire 13B.

Next, the top protective layer 15A produced in the step of the item (3) was laminated onto the top side of the dielectric layer 11, on which the top electrode layer 12A and the top conductive wire 13A were formed, to cover the whole of the top electrode layer 12A and one portion of the top conductive wire 13A.

Furthermore, copper foil pieces were fitted to respective ends of the top conductive wire 13A and the bottom conductive wire 13B to form a top connecting portion 14A and a bottom connecting portion 14B (see FIG. 11C). Thereafter, lead wire 18, which were to be external conductive wires, were fixed to the top connecting portion 14A and the bottom connecting portion 14B by soldering.

Thereafter, a PET film 17 of 100 µm thickness was bonded through an acrylic adhesive tape 16 (Y-4905 (thickness: 0.5 mm), manufactured by the company 3M) onto the top connecting portion 14A and the bottom connecting portion 14B at their positions over the bottom protective layer 15B to reinforce the moieties (see FIG. 11D). In this way, a sensor body 10 was finished.

### Production of Capacitive Sensor A (Adhesive-Type Capacitive Sensor)

The capacitive sensor 202 illustrated in FIGS. 6A and 6B was produced in the following manner.

Initially, a stretchable polyester cloth piece (KKF 5550, Uni Textile Co., Ltd.) was cut into two pieces each having a size of 75 mm x 20 mm. Furthermore, both corners of one of short sides of each of the two were each cut out by a size of 5 mm x 5 mm. In this way, corner-cut-out pieces, the number of which was two, were prepared. Next, a stretchable adhesive agent was used to bond the two stretchable polyester cloth pieces to each other to sandwich one of the sensor bodies 10 between these stretchable polyester cloth pieces. In this way, a covering member 221 was formed.

Next, an adhesive agent (TECHNOGEL HIT-B3R75W (trade name)), manufactured by Sekisui Plastic Co., Ltd.) was used to form an adhesive layer 223 of 60 mm x 15 mm size onto one of the two surfaces of the covering member 221.

Lastly, connecting terminals were fitted to ends of the lead wire 18 that were opposite to the sensor body 10 to form a connecting member 222. In this way, a capacitive sensor A was finished.

### Production of Capacitive Sensor B (Ring-Type Capacitive Sensor)

The capacitive sensor 2 illustrated in FIG. 2 was produced by the following method.

Initially, a stretchable cloth piece (SUPER STRETCH II, a company Yuzawaya) was cut to prepare two cloth pieces each having a size of 18 cm x 2 cm. A stretchable adhesive agent was used to bond the two cloth pieces to each other to sandwich another of the sensor bodies 10 between the stretchable cloth pieces. In this way, a covering member 21 was formed.

Next, rubbery strings (Sewed-Rubber No. 59, Daiso Industries Co., Ltd.) 23A each having a length of 30 cm and a width of 0.8 cm were fixed, respectively, to both ends of the covering member 21. Furthermore, an adjustor (stopper) 23B was fitted to the rubbery strings at their respective free end sides.

Lastly, connecting terminals were fitted to ends of the lead wire 18 that were opposite to the sensor body 10 to form a connecting member 22. In this way, a capacitive sensor B was finished.

### Swallowing Movement Measuring Devices

Each of the capacitive sensors A and B was fitted through the connecting member to an instrument Power Lab 16/35, PL3516 (manufactured by AD Instruments Ltd.) to produce a swallowing movement measuring device.

Each of the swallowing movement measuring devices was used to measure an examinee's swallowing movement.

At this time, measuring conditions of the Power Lab 16/35, PL3516 were set as follows: sampling frequency: 100 Hz; measuring range: ±5 V; indicating software: Lab Chart 8.0.9.

Signal treatment conditions were set as follows:
Quaternary Butterworth filter,
Low-pass filtering, and
Cut off: 1 Hz.

### Example 1: Measurement of laryngeal Movement in Usual Swallowing

When the examinee (healthy adult man) swallowed, a change of the capacitance of the sensor was measured by a method described below.

The capacitive sensor A was fitted to the body skin of the examinee's laryngeal part.

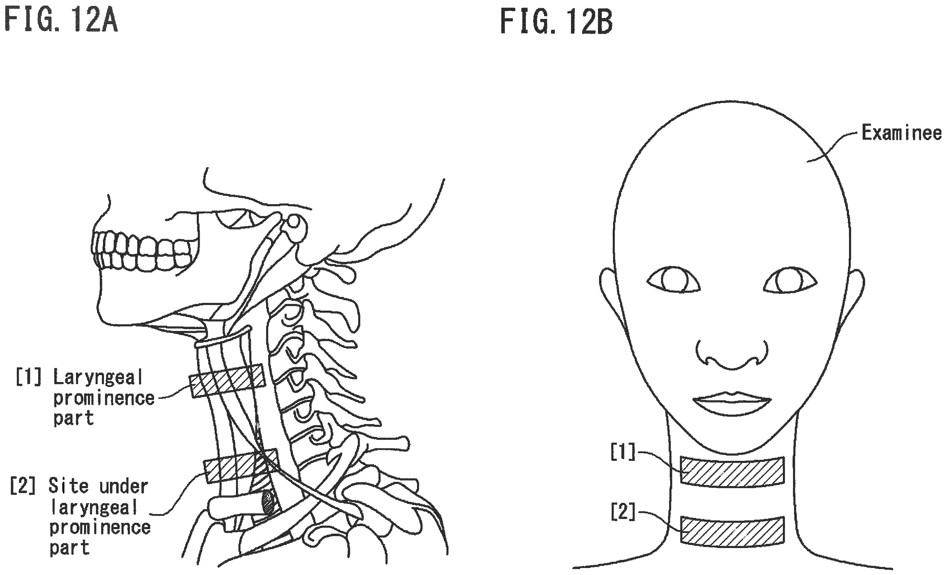

As illustrated in FIGS. 12A and 12B, at this time, the capacitive sensor A (sensor body 10) was bonded to the examinee to bring the capacitive sensor A into contact with the examinee at his positions corresponding to his laryngeal prominence part [1] and to his site [2] under the laryngeal prominence part.

### Next, the examinee was caused to swallow a liquid (1) (3 mL of water) and a semi-solid (3 g of jelly) as food boluses, and then the capacitance of the sensor was measured in accordance with steps described below.

Simultaneously, a vertical movement of the examinee's larynx was visually observed when the examinee swallowed.

### Swallowing Steps

1. The examinee held each of the food boluses on his mouth floor and then took a designated pose.
2. A measurement by use of the swallowing movement measuring device was started in synchronization with a Metronome (1 Hz).
3. At a second clap of the Metronome (after two seconds), the examinee swallowed the food bolus.
4. After eight seconds, the measurement of the capacitance was ended.

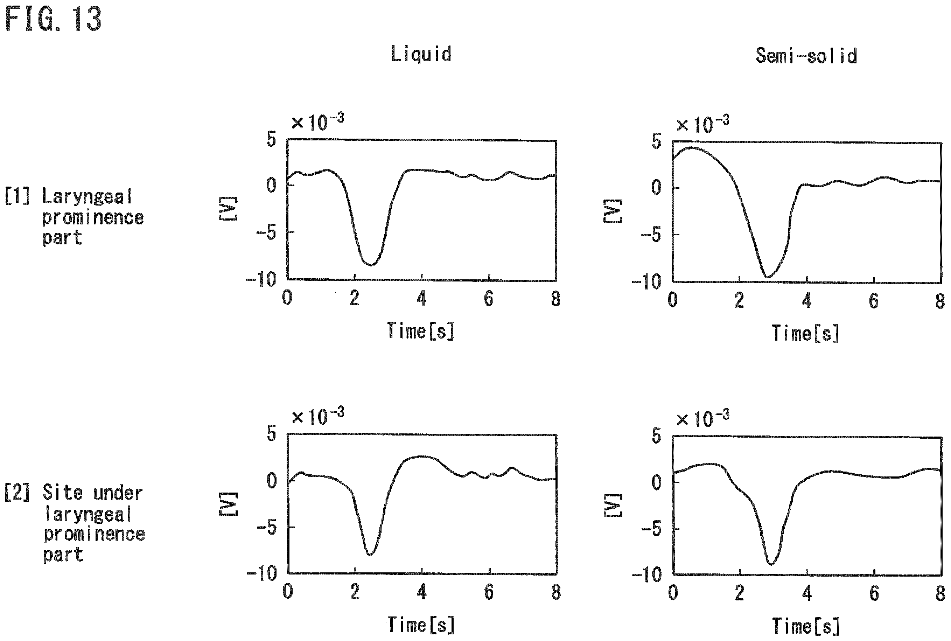

As a result, as illustrated in FIG. 13, about each of the liquid and the semi-solid, in the measurement of each of his laryngeal prominence part [1] and his site [2] under the laryngeal prominence part, a capacitance change was measured. This change was a change in which the following waveform was drawn: a V-shaped waveform such that the sensor output was once lowered and then restored to its original state.

It appeared that this V-shaped waveform was correlative with a visually-observed vertical movement of his larynx to reflect the movement of his larynx that followed the swallowing.

### Example 2: Measurement (1) of Laryngeal Movement Based on Mendelson's Technique in Swallowing

The Mendelson's technique is a method which can be expected, as a manner alternative for swallowing, to produce an advantageous effect of decreasing pharyngeal residues and misswallowing.

Specifically, when examinee's larynx raise-up and pharyngeal constriction each reach a peak, an instruction that the examinee should temporarily stop swallowing is given to the examinee. This state is kept over two or three seconds, or five or six seconds. Subsequently, the examinee relaxes, and further expires air and returns to the examinee's state before the swallowing.

In the present example, an examinee swallowed according to the Mendelson's technique. At this time, the movement of the examinee' larynx was measured.

Initially, the capacitive sensor A (sensor body 10) was fitted to the examinee (healthy adult man) to bring the capacitive sensor A into contact with the examinee at his position corresponding to his laryngeal prominence part [1].

Next, the examinee was caused to swallow a liquid (3 mL of water) as a food bolus, and then the capacitance of the sensor was measured in accordance with steps described below. The measurement was made three times. The following instruction was beforehand given to the examinee: an instruction that the examinee should try to swallow water at a time, and keep a state that his throat is raised up for two seconds.

Simultaneously, a vertical movement of the examinee's larynx was visually observed when the examinee swallowed.

### Swallowing Steps

1. The examinee poured a liquid on his mouth floor and then took a designated pose.
2. A measurement by use of the swallowing movement measuring device was started in synchronization with a Metronome (1 Hz).
3. At a second clap of the Metronome (after two seconds), the examinee turned into the state of raising up his throat (the larynx raise-up was kept). This sate was kept over two claps of the Metronome (two seconds), and the examinee subsequently swallowed the liquid.
4. After eight seconds, the measurement of the capacitance was ended.

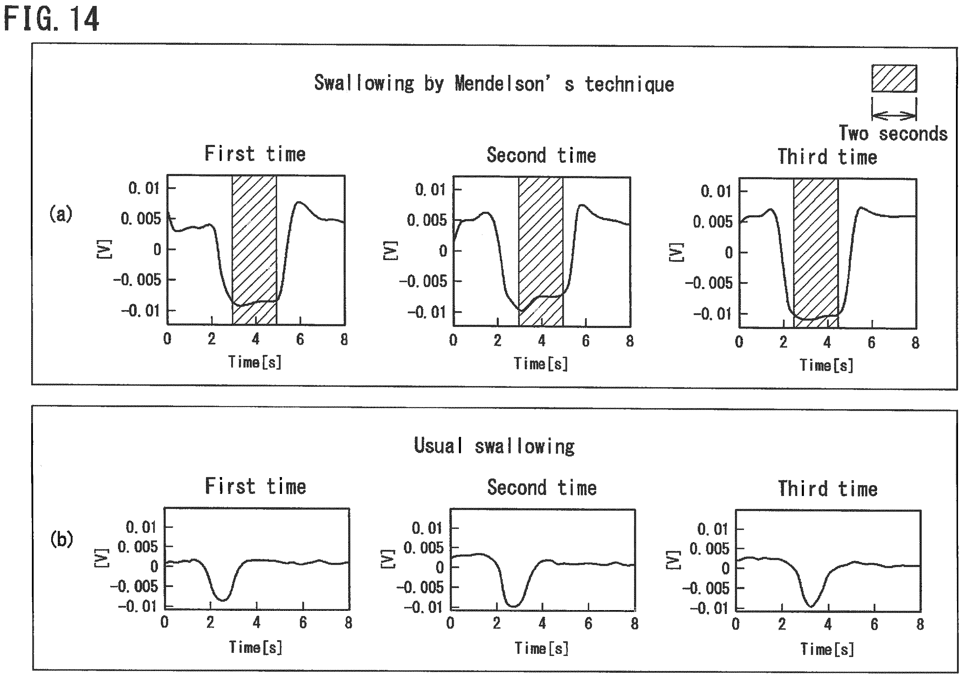

As a result, a capacitance change as shown in FIG. 14A was measured.

According to this result, a period (approximately two seconds) when a valley moiety of a V-shaped waveform was extended was substantially consistent with a temporary-stop keeping period (the two claps = two seconds) when the raise-up of the examinee's larynx reached a peak. This result was also consistent with the visually observed result.

The same examinee usually swallowed 3 mL of water (see the swallowing steps in Example 1). FIG. 14B shows measured results of the capacitance in this swallowing case. In this case, the same V-shaped waveform as in Example 1 was measured.

These results have made it evident that the measured results in FIG. 14A reflect the examinee's swallowing movement according to the Mendelson's technique.

### Example 3: Measurement (2) of Laryngeal Movement Based on Mendelson's Technique in Swallowing

The movement of an examinee's larynx according to the Mendelson's technique was measured in the same way as in Example 2 except that the capacitive sensor B was used instead of the capacitive sensor A.

In the present example, the capacitive sensor B (sensor body 10) was fitted to the examinee (healthy adult man) to bring the capacitive sensor B into contact with the examinee at his position corresponding to his laryngeal prominence part [1].

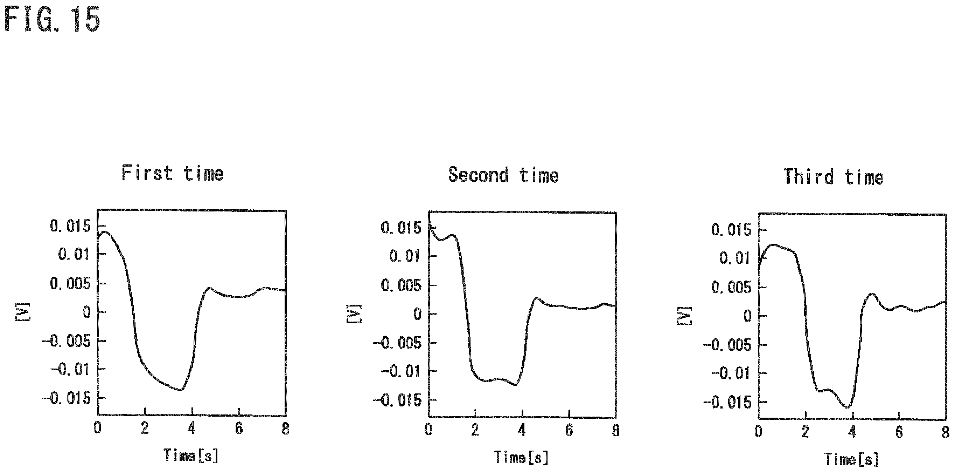

As a result, a capacitance change as shown in FIG. 15 was measured.

Results shown in FIG. 15 were also substantially the same results as in Example 2 to give the following results: a period (approximately two seconds) when a valley moiety of a V-shaped waveform was extended was substantially consistent with a temporary-stop keeping period (the two claps = two seconds) when the raise-up of the examinee's larynx reached a peak.

From this matter, it has been made evident that in the same manner as the capacitive sensor A, the capacitive sensor B is also a capacitive sensor capable of an examinee's swallowing movement according to the Mendelson's technique.

### Production of Sensor Body C

A sensor body C having substantially the same structure as the sensor body 50 illustrated in FIGS. 8A and 8B was produced. The sensor body C was produced basically in the same manner as used to produce the sensor body.

### (1) Production of Dielectric Layer

Using a method equivalent to the method adopted in the item (1) in the production of the sensor body A, a sheet was produced which was made of a polyether-based urethane elastomer. Thereafter, the resultant urethane sheet was cut out into a piece having a size of 56 mm x 70 mm x 100 µm thickness. Thereafter, about one of the short sides of the cut-out sheet piece, at its seventh positions, pieces each having a size of 4 mm x 4 mm x 100 µm were cut out at intervals of 8 mm from one of its corners along the short side to produce a dielectric layer 51.

### (2) Preparation of Dielectric Layer Material

A carbon nanotube dispersed liquid was yielded in the same way as in the item (2) in the production of the sensor body A.

### (3) Production of Protective Layers

Using the same manner as in the item (1) except that the thickness of the sheet was changed, the following were produced: a bottom protective layer 55B made of the polyether-based urethane elastomer and having a size of 56 mm x 70 mm x 50 µm; and a top protective layer 55A made of the same elastomer and having a size of 56 mm x 63 mm x 50 µm.

### (4) Production of Sensor Body

Initially, a mask (not illustrated) made of a PET film subjected to release-treatment was caused to adhere onto a single surface (top surface) of the bottom protective layer 55B produced in the step of the item (3).

In the mask, plural opening corresponding to the position of the bottom electrode layer and the bottom conducting wire is formed. The size of the each opening is such that the portion corresponding to the bottom electrode layer has a size of 5 mm in width × 54 mm in length, and the portion corresponding to the bottom conducting wire has a size of 1.5 mm in width × 6 mm in length.

Next, an air brush was used to paint 37.4 g of the carbon nanotube dispersed liquid prepared in the step of the item (2) onto the workpiece. Thereafter, the workpiece was dried at 100 °C for 10 minutes to form bottom electrode layers 52B1 to 52B7, and bottom conductive wires 53B1 to 53B7. The mask was then peeled away.

Next, the dielectric layer 51 was laminated onto the bottom protective layer 55B. In this step, the dielectric layer 51, which was produced in the step of the item (1), was bonded onto the bottom protective layer 55B to cover the whole of the bottom electrode layers 52B1 to 52B7 and partial regions of the bottom conductive wires 53B1 to 53B7.

Furthermore, top electrode layers 52A1 to 52A7, and top conductive wires 53A1 to 53A7 were formed on the top side of the dielectric layer 51. The formation of the top electrode layers 52A1 to 52A7, and the top conductive wires 53A1 to 53A7 was attained in the same way as used to form the bottom electrode layers 52B1 to 52B7, and the bottom conductive wires 53B1 to 53B7.

Next, the top protective layer 55A formed in the step of the item (3) was laminated onto the top side of the dielectric layer 51, on which the top electrode layers 52A1 to 52A7, and the top conductive wires 53A1 to 53A7 were formed, to cover the whole of the top electrode layers 52A1 to 52A7, and partial regions of top conductive wires 53A1 to 53A7.

Furthermore, copper foil pieces were fitted, respectively, to individual ends of the top conductive wires 53A1 to 53A7, and the bottom conductive wires 53B1 to 53B7 to form top connecting portions 54A1 to 54A7, and bottom connecting portions 54B1 to 54B7.

Thereafter, lead wire (not illustrated) which were to be external conductive wires were fixed to the top connecting portions 54A1 to 54A7 and the bottom connecting portions 54B1 to 54B7 by soldering.

Next, a PET film of 100 µm thickness was bonded through an acrylic adhesive tape (Y-4905 (thickness: 0.5 mm), manufactured by the company 3M) onto the top connecting portions 54A1 to 54A7 and the bottom connecting portions 54B1 to 54B7 at their positions over the bottom protective layer 55B. Through these steps, a sensor body 50 was finished.

### <Production of Capacitive Sensor C>

Initially, a stretchable polyester cloth piece (KKF 5550, Uni Textile Co., Ltd.) was cut out to prepare two cloth pieces each having a size of 85 mm x 62 mm.

Next, a stretchable adhesive agent was used to bond the two stretchable polyester cloth pieces to each other to sandwich the sensor body 50 between these stretchable polyester cloth pieces. In this way, a covering member 321 was formed.

Apart from this member, prepared was a cut non-stretchable cloth piece having a size of 70 mm x 70 mm. One corner of this cloth piece was cut out into a size of 40 mm x 55 mm. In this way, an L-shaped cloth piece was prepared. This cloth piece, and the same cloth pieces were prepared in a total number of four.

Furthermore, two strings (mask rubber, Yuzawaya Shoji Co., ltd.) each cut into a length of 440 mm were prepared.

Next, both ends in the longitudinal direction (right and left direction in FIG. 7) of the sensor body 50, in which the covering members 321 were located, respectively, on its both surfaces, were each sandwiched between two of the L-shaped cloth pieces to form linking moieties 323C.

Furthermore, each of the strings was passed through an adjustor (cord stopper) 325, and then fitted to one of the linking moieties 323C. In this way, each first fitting portion 323A was formed. Moreover, a hook and loop fastener (for sewing ECOMAGIC, manufactured by Kuraray Fastening Co., Ltd.) cut into a size of 15 mm x 200 mm was sewed with each of the linking moieties 323C. In this way, each second fitting portion 323B was formed.

Lastly, connecting terminals were fitted, respectively, to ends of the lead wire that were opposite to the sensor body 50 to form connecting members. In this way, a capacitive sensor C was finished.

### Swallowing Movement Measuring Device

The capacitive sensor C was fitted through the connecting members to an instrument Power Lab 16/35, PL3516 (manufactured by AD Instruments Ltd.) to produce a swallowing movement measuring device.

This swallowing movement measuring devices was used to measure an examinee's swallowing movement.

At this time, measuring conditions were set as follows: 100 Hz, and a range of ±5 V. In a data processing in Example 4 described below, the average of 10 data was used. This case is substantially equivalent to the case of measurement at 10 Hz.

### Example 4: Measurement of Laryngeal Movement in Usual Swallowing

When an examinee (healthy adult man) swallowed, a change of the capacitance of the sensor was measured by a method described below.

The capacitive sensor C was fitted to the body skin of the examinee's laryngeal part.

As illustrated in FIGS. 9A and 9B, at this time, the capacitive sensor C (sensor body 10) was bonded to the examinee to cover the entire body skin of his laryngeal part. In more detail, the capacitive sensor was fitted thereto to satisfy the following states (1) and (2):
(1) The detecting portion (sequence 3 described below) where the rectangular electrode 52A3 and the rectangular electrode 52B3 were opposite to each other, and the detecting portion (sequence 4 described below) where the rectangular electrode 52A4 and the rectangular electrode 52B4 were opposite to each other were located at positions of the device that corresponded to his laryngeal prominence part.
(2) The detecting portion (sequence 1 described below) where the rectangular electrode 52A1 and the rectangular electrode 52B1 were opposite to each other was located at a position of the device that corresponded to his site under his laryngeal prominence part.

The examinee was caused to swallow saliva at a time. At this time, respective capacitance changes in the detecting portions were individually measured at the detecting portions.

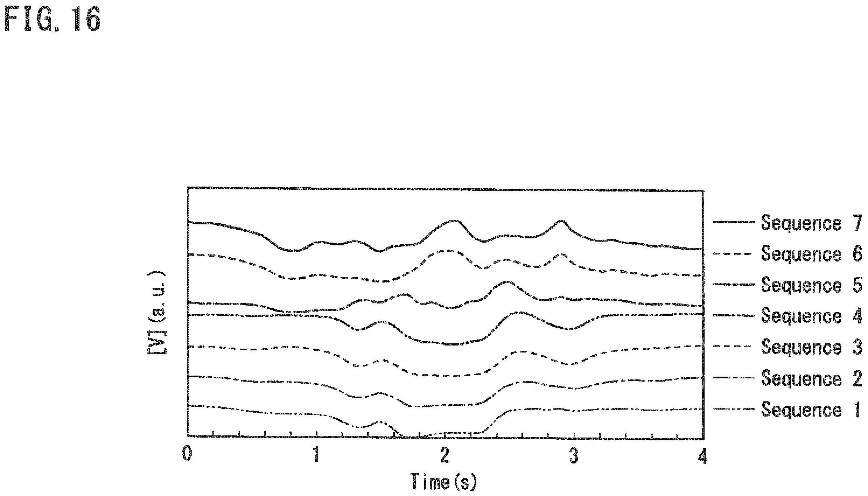

The results are shown in FIG. 16. In a graph shown in FIG. 16, the respective measured results of the detecting portions are shown as sequences 1 to 7 in an order from the bottom of this figure. In other words, sequence 1 is a measured value of the detecting portion where the rectangular electrode 52A1 and the rectangular electrode 52B1 were opposite to each other, and sequence 7 is a measured value of the detecting portion where the rectangular electrode 52A7 and the rectangular electrode 52B7 were opposite to each other.

The graph shown in FIG. 16 is a graph in which in order to make respective results of the individual sequences easy to see, the results are located in the direction of a vertical axis of this figure in the state of making their time axes consistent with each other. Vertical positions of the results in the vertical axis direction do not show the magnitude of the measured values.

It has been considered from the results shown in FIG. 16 that in the case of using a capacitive sensor having a sensor body having plural rectangular electrodes located side by side to be separated from each other, measurements can be made about the speed of the vertical movement of an examinee's laryngeal prominence part and the shift range of the movement.

### LIST OF REFERENCE SIGNS

- 1: Swallowing movement measuring device
- 2, 102: Capacitive sensors
- 202, 302: Capacitive sensors
- 3: Measuring unit
- 3a: Schmitt trigger oscillation circuit
- 3b: F/V conversion circuit
- 4: Indicator
- 4a: Monitor
- 4b: Arithmetic circuit
- 4c: Memory
- 10, 50: Sensor bodies
- 11, 51: Dielectric layers
- 12A, 52A: Top electrode layers (first electrode layers)
- (52A1 to 52A7): Top electrode layers (first electrode layers)
- 12B, 52B: Bottom electrode layers (second electrode layers)
- (52B1 to 52B7): Bottom electrode layers (second electrode layers)
- 13A, 53A: Top conductive wires
- (53A1 to 53A7): Top conductive wires
- 13B, 53B: Bottom conductive wires
- (53B1 to 53B7): Bottom conductive wires
- 14A, 54A: Top connecting portions
- (54A1 to 54A7): Top connecting portions
- 14B, 54B: Bottom connecting portions
- (54B1 to 54B7): Bottom connecting portions
- 15A, 55A: Top protective layers
- 15B, 55B: Bottom protective layers
- 21, 121: Covering members
- 221, 321: Covering members
- 22, 122: Connecting members
- 222, 322: Connecting members
- 23, 123, 323: Fitting members
- 30: Forming apparatus
- 223: Adhesive layer

## Claims

1. A swallowing movement measuring device for measuring an examinee's swallowing movement on the basis of a movement of the examinee's larynx when the examinee swallows a bolus;
the device comprising a capacitive sensor to be brought into contact with a body skin of the examinee's laryngeal part, and a measuring unit that measures a change of the capacitive sensor in capacitance;
wherein the capacitive sensor comprises a sensor body comprising sheet-like dielectric layer, a first electrode layer and a second electrode layer,
the first and the second electrode layers are formed on a top surface and a bottom surface of the dielectric layer respectively, and are at least partially opposed to each other across the dielectric layer,
the at least partially opposed portions of the first and the second electrode layers constitute a detection portion, and
the sensor body is reversibly deformable to change areas of the top and bottom surfaces of the dielectric layer.

2. The swallowing movement measuring device according to claim 1,
wherein the dielectric layer comprises an elastomer composition.

3. The swallowing movement measuring device according to claim 1 or 2,
wherein the sensor body has a plurality of the detecting portions.

4. The swallowing movement measuring device according to claim 3,
wherein the first electrode layer and the second electrode layer each comprise a plurality of rectangular electrodes arranged side by side to be separated from each other, and
the rectangular electrodes included in the first electrode layer and the rectangular electrodes included in the second electrode layer are arranged to be overlapped with each other in a thickness direction of the dielectric layer.

5. The swallowing movement measuring device
according to any one of claims 1 to 4,
wherein the capacitive sensor further comprises a fitting member; and wherein the fitting member is formed in such a manner that the capacitive sensor can be brought into contact with the body skin of the examinee's laryngeal part under a condition of rendering a state that the detecting portion or the detecting portions are stretched an initial state.

6. A method for measuring a swallowing movement, comprising:
- bringing a capacitive sensor into contact with a body skin of an examinee's laryngeal part;
- measuring a change of the capacitive sensor in capacitance, the change being based on a movement of the examinee's larynx when the examinee swallows a food bolus; and
- measuring the examinee's swallowing movement on the basis of a result of the measurement;
wherein the capacitive sensor comprises a sensor body comprising sheet-like dielectric layer; a first electrode layer and a second electrode layer,
the first and the second electrode layers are formed on a top surface and a bottom surface of the dielectric layer respectively, and are at least partially opposed to each other across the dielectric layer,
the at least partially opposed portions of the first and the second electrode layers constitute a detection portion, and
the sensor body is reversibly deformable to change areas of the top and bottom surfaces of the dielectric layer.

7. The method for measuring the swallowing movement according to claim 6,
wherein the dielectric layer comprises an elastomer composition.

8. The method for measuring the swallowing movement according to claim 6 or 7,
wherein the sensor body has a plurality of the detecting portions.

9. The method for measuring the swallowing movement according to claim 8,
wherein the first electrode layer and the second electrode layer each comprise a plurality of rectangular electrodes arranged side by side to be separated from each other, and
wherein the rectangular electrodes included in the first electrode layer and the rectangular electrodes included in the second electrode layer are arranged to be overlapped with each other in a thickness direction of the dielectric layer.

10. The method for measuring the swallowing movement
according to any one of claims 6 to 9,
wherein the capacitive sensor is brought into contact with the body skin of the examinee's laryngeal part under a condition of rendering a state that the detecting portion or the detecting portions are stretched an initial state.

11. The method for measuring the swallowing movement
according to any one of claims 6 to 10,
wherein the capacitive sensor is brought into contact with of a laryngeal prominence part of the body skin of the laryngeal part.
